# FASCICULE DE BREVET EUROPEEN

(11) **EP 4 344 729 B1**
(45) Date de publication et mention de la délivrance du brevet: **25.06.2025**
(21) Numéro de dépôt: 23198137.4
(22) Date de dépôt: 19.09.2023
(51) Int. Cl.: A61N 5/06, A61B 5/00

(54) **PROCÉDÉ ET DISPOSITIF DE DÉTERMINATION DE L'ÉTAT DE FONCTIONNEMENT D'UN IMPLANT LUMINEUX**
VERFAHREN UND VORRICHTUNG ZUR BESTIMMUNG DES BETRIEBSZUSTANDES EINES LICHTIMPLANTATS
METHOD AND DEVICE FOR DETERMINING THE OPERATING STATE OF A LIGHT IMPLANT

(30) Priorité: 29.09.2022 FR 2209903
(43) Date de publication de la demande: 03.04.2024
(73) Titulaire: Commissariat à l'Energie Atomique et aux Energies Alternatives, 75015 Paris (FR); Centre Hospitalier Universitaire Grenoble Alpes, 38700 La Tronche (FR); Université Grenoble Alpes, 38400 Saint-Martin-d'Hères (FR)
(72) Inventeur: BLEUET, Pierre, 38054 Grenoble cedex 09 (FR); CHABROL, Claude, 38054 Grenoble cedex 09 (FR); MORO, Cécile, 38054 Grenoble cedex 09 (FR); CHABARDES, Stephan, 38700 LA TRONCHE (FR); BENABID, Alim Louis, 38054 Grenoble cedex 09 (FR)
(74) Mandataire: INNOV-GROUP

(56) Documents cités:
- US-A1- 2014 288 386
- US-A1- 2016 007 851
- US-A1- 2018 242 840
- US-A1- 2021 178 175
- US-B1- 11 395 620

## Description

### Domaine technique de l'invention

La présente invention se rapporte à un procédé de détermination de l'état de fonctionnement d'un implant lumineux. L'invention se rapporte également à un dispositif de diagnostic du fonctionnement de l'implant lumineux.

### Etat de la technique

Récemment, il a été proposé de ralentir la progression d'une pathologie en utilisant la lumière (tout rayonnement électromagnétique qui va de l'ultraviolet à l'infrarouge lointain en passant par le visible). Ce principe est notamment employé sous la forme d'un implant cérébral, intracrânien, comprenant une source lumineuse chargée de venir éclairer une zone du cerveau en vue de traiter la pathologie. L'implant peut notamment se présenter sous la forme d'une fibre optique à l'extrémité de laquelle la lumière est diffusée.

Les demandes de brevets US 2014/288386 A1, US 2021/178175 A1, EP3302687A1, EP3723851A1 et EP3834884A1 décrivent de telles sondes intracrâniennes.

Cependant, une fois que ces sondes sont implantées, il est difficile de savoir si la lumière est bien diffusée en bout de sonde. En effet, la source lumineuse peut être défectueuse, la fibre optique peut être cassée ou mal implantée. Il en ressort que l'état de fonctionnement de l'implant est parfois difficile à connaître, sans avoir à le retirer pour démontage et vérification.

Il existe donc un besoin de disposer d'une solution simple pour déterminer l'état de fonctionnement d'un implant lumineux intracrânien tel que décrit dans les documents de l'état de la technique.

Le but de l'invention est de proposer une solution technique permettant de remplir cet objectif.

### Exposé de l'invention

Ce but est atteint par un procédé de détermination de l'état de fonctionnement d'un implant lumineux implanté dans le cerveau d'un être vivant, ledit implant lumineux comportant une source lumineuse chargée d'émettre de la lumière dans le cerveau de l'être vivant, ledit procédé utilisant un dispositif de diagnostic qui comporte un récepteur d'un signal lumineux transmis à travers un premier œil de l'être vivant et des moyens de détermination de l'état de fonctionnement de l'implant lumineux à partir du signal lumineux transmis reçu,

Ledit procédé comportant :
- Une étape de positionnement du récepteur du dispositif de diagnostic en vis-à-vis du premier œil de l'être vivant,
- Une étape de mesure d'un signal lumineux reçu par le récepteur lorsque l'implant lumineux est activé,
- Une étape de comparaison entre les données représentatives du signal lumineux reçu par le récepteur et au moins une donnée de référence,
- Une étape de détermination de l'état de fonctionnement de l'implant lumineux entre un état fonctionnel et un état non-fonctionnel en tenant compte du résultat de l'étape de comparaison.

Selon une particularité, ladite donnée de référence est pré-mémorisée ou acquise lors d'une étape de calibration du dispositif de diagnostic.

Selon une autre particularité, l'étape de calibration consiste en une étape de mesure d'un signal lumineux de référence, réalisée lorsque l'implant lumineux est désactivé.

Selon une autre particularité, l'étape de détermination de l'état de fonctionnement de l'implant lumineux consiste à déterminer au moins un écart entre ladite donnée de référence et une donnée desdites données représentatives du signal lumineux reçu et à comparer ledit écart avec une valeur seuil prédéterminée.

Selon une autre particularité, le procédé comporte une étape de positionnement d'une source de lumière ponctuelle en vis-à-vis d'un deuxième œil de l'être vivant et une étape d'activation de ladite source de lumière ponctuelle.

L'invention concerne également un dispositif de diagnostic utilisé pour mettre en œuvre le procédé tel que défini ci-dessus, le dispositif comportant un support mécanique sur lequel est fixé ledit récepteur et des moyens de détermination de l'état de fonctionnement dudit implant lumineux, connectés audit récepteur et configurés pour traiter des données en provenance dudit récepteur.

Selon une réalisation particulière, le récepteur est une caméra.

Selon une autre réalisation particulière, le récepteur comporte une ou plusieurs photodiodes.

Selon une particularité, le dispositif comporte une source de lumière ponctuelle fixée audit support et destinée à être arrangée en vis-à-vis d'un deuxième œil de l'être vivant, en parallèle du récepteur.

Selon une réalisation particulière, le support mécanique comporte un plateau optique muni d'une mentonnière et sur lequel est fixé ledit récepteur.

Selon une autre réalisation particulière, le support mécanique comporte un masque à placer autour de la tête de l'être-vivant.

Selon une particularité, le masque comporte deux lunettes, une première lunette accueillant ledit récepteur et une deuxième lunette accueillant une source de lumière ponctuelle.

### Brève description des figures

D'autres caractéristiques et avantages vont apparaître dans la description détaillée qui suit faite en regard des dessins annexés dans lesquels :
- La figure 1 illustre le principe de fonctionnement d'un implant lumineux utilisé dans le cadre de l'invention et celui du dispositif de diagnostic de l'invention ;
- La figure 2 représente un premier exemple de réalisation du dispositif de diagnostic conforme à l'invention ;
- La figure 3 représente un deuxième exemple de réalisation du dispositif de diagnostic conforme à l'invention ;

### Description détaillée d'au moins un mode de réalisation

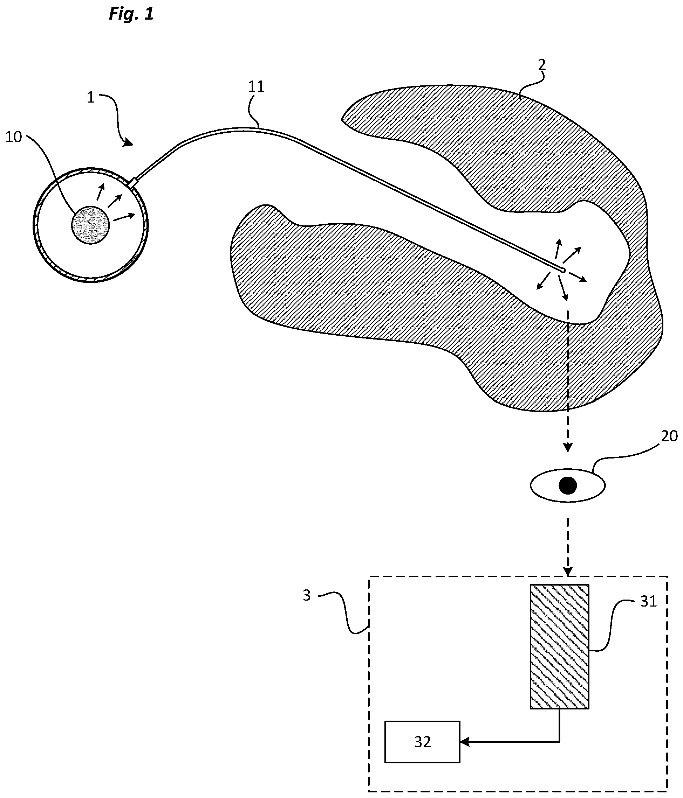

En référence à la figure 1, l'invention vise un dispositif 3 de diagnostic du fonctionnement d'un implant lumineux 1 destiné à être implanté dans le cerveau 2 d'un être vivant. Lorsqu'il fonctionne correctement, cet implant lumineux 1 est destiné à émettre de la lumière à destination d'au moins une zone du cerveau 2 de l'être vivant. Par lumière ou signal lumineux, on entend tout rayonnement électromagnétique qui va de l'ultraviolet à l'infrarouge lointain en passant par le visible.

L'implant 1 utilise au moins une source lumineuse 10 et comporte une sonde 11 par laquelle la lumière est diffusée. La sonde 11 peut se présenter sous la forme d'une fibre optique chargée d'acheminer la lumière de la source lumineuse 10 jusqu'à son extrémité distale, située à proximité des tissus à traiter. Le principe de réalisation de l'implant peut être varié. Les demandes de brevets EP3302687A1, EP3723851A1 et EP3834884A1 décrivent de telles sondes intracrâniennes. Par le terme implant, on, entend que le dispositif comporte au moins une partie implantée dans le cerveau de l'être vivant, même s'il peut éventuellement comporter des parties situées à l'extérieur du corps de l'être vivant.

Selon un aspect particulier de l'invention, le dispositif 3 de diagnostic comporte :
- Eventuellement un support mécanique ;
- Un récepteur 31 d'un signal lumineux transmis à travers l'œil de l'être vivant, fixé audit support mécanique ;
- Des moyens 32 de détermination de l'état de fonctionnement de l'implant lumineux ;

L'objectif du dispositif 3 de diagnostic est de déterminer l'état de fonctionnement de l'implant lumineux 1 placé à l'intérieur du crâne de l'être vivant en détectant la présence ou l'absence d'un signal lumineux S transmis à travers l'œil 20 de l'être vivant. Le dispositif 3 de diagnostic présente la particularité d'être non invasif, c'est-à-dire qu'il est placé à l'extérieur du corps de l'être vivant.

Le récepteur 31 est configuré pour collecter les photons émis par l'implant lumineux 1 et qui sont diffusés par les tissus et ressortent via le globe oculaire.

Différentes variantes de réalisation de récepteur peuvent être envisagées :
- Un détecteur utilisant une ou plusieurs photodiodes,
- Une caméra très sensible.

Dans tous les cas, le récepteur 31 doit permettre de mesurer des puissances optiques très faibles (entre le femtoWatt et le nanoWatt) et avec des temps d'intégration suffisamment courts (de l'ordre de la minute ou moins) pour que l'examen soit compatible avec une mesure sur un être vivant.

De manière non limitative, le récepteur 31 peut notamment être un détecteur à comptage de photons multi-pixels commercial, basé sur des photodiodes à avalanche en mode Geiger, ou d'une photodiode silicium de large diamètre permettant un fort angle solide de détection. Ce type de détecteur ne fournit pas une image, mais une valeur ponctuelle ; cependant leur sensibilité peut être largement supérieure à celle d'une caméra, et ils sont plus compacts et plus rapides. Il est possible d'ajouter une lentille à large ouverture numérique devant le détecteur, pour collecter le plus de photons. Cette lentille joue alors le rôle de condenseur.

Selon un aspect particulier de l'invention, l'observation doit être réalisée avec un minimum de lumière parasite. Autrement dit, l'observation se fait avantageusement dans des conditions d'obscurité totale pour éviter toute pollution lumineuse due à l'environnement. A titre d'exemple et de manière non limitative, le niveau de lumière ambiant doit être d'au moins un ordre de grandeur inférieur à celui du signal lumineux transmis à travers l'œil.

Selon un aspect particulier de l'invention, les moyens 32 de détermination de l'état de fonctionnement de l'implant lumineux peuvent comporter un microprocesseur sur lequel vient se connecter le récepteur 31. Ils sont chargés d'interpréter les données représentatives des signaux reçus par le récepteur. Différents modes de traitement peuvent être envisagés pour déterminer l'état de fonctionnement de l'implant lumineux :
Premier mode de traitement :
   - Il peut s'agir de récupérer un premier signal lumineux de référence, implant lumineux désactivé, et de mémoriser une ou plusieurs données de référence représentatives de ce premier signal lumineux ; Cette mesure est avantageusement réalisée dans l'obscurité totale ;
   - Ensuite, après activation de l'implant, on mesure un deuxième signal lumineux au niveau du récepteur 31 ;
   - On compare une ou plusieurs des données représentatives du deuxième signal lumineux avec une ou plusieurs des données de référence correspondantes ;
Deuxième mode de traitement :
   - Une ou plusieurs données de référence sont pré-mémorisées en usine et correspondent à un état de référence dans lequel le système est placé dans l'obscurité totale ;
   - Le reste du traitement est identique à celui décrit ci-dessus ;

Par données représentatives d'un signal lumineux, on entend une intensité lumineuse maximale, par exemple prise au niveau d'un ou plusieurs pixels de l'image capturée (par la caméra), une moyenne de plusieurs intensités lumineuses mesurées... Toute autre donnée pourrait être envisagée.

Si les données comparées sont identiques, les moyens 32 de détermination de l'état de fonctionnement d'un implant lumineux 1 peuvent en conclure que l'implant lumineux 1 est défectueux. Si une ou plusieurs des données issues du deuxième signal reçu diffèrent de celles représentatives du premier signal, les moyens 32 de détermination peuvent en conclure que l'implant lumineux 1 est fonctionnel. Il est possible de régler un seuil au-dessus duquel les moyens 32 de détermination de l'état de fonctionnement de l'implant déterminent qu'une ou plusieurs des données issues du deuxième signal diffèrent suffisamment de celles représentatives du premier signal.

De manière non limitative, un mode de traitement simple est le suivant :
- Une première image, implant lumineux 1 intracérébral éteint, permet de vérifier les conditions d'obscurité : le signal ou l'image mesurée ne corresponde qu'au bruit du détecteur ou de la caméra ; Ensuite,
- Une deuxième image, implant lumineux 1 activé, permet, si l'implant lumineux 1 est parfaitement fonctionnel, de mettre en évidence la contribution au signal ou à l'image mesurée des photons issus de l'implant intracérébral et diffusés dans les tissus.

D'autres modes de fonctionnement peuvent bien entendu être envisagés, selon les moyens disponibles et mis en œuvre.

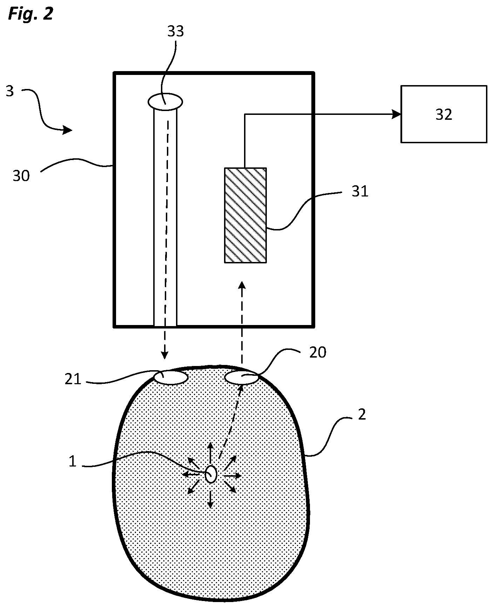

Dans un premier mode de réalisation illustré par la figure 2, le support mécanique se présente sous la forme d'un plateau 30 optique sur lequel on vient fixer le récepteur 31, par exemple la caméra, en vis-à-vis de l'un des deux yeux (par exemple l'œil 20) de l'être vivant. Le plateau 30 optique peut être mobile sur plusieurs axes afin de pouvoir adapter la position de la caméra en vis-à-vis de l'œil ciblé. Il peut être muni d'une mentonnière (non représentée) pour stabiliser la tête de l'être vivant en vis-à-vis du système.

Avantageusement, sur ce plateau 30 optique, on viendra positionner une source de lumière 33 ponctuelle devant l'autre œil 21, pour faire en sorte que l'être vivant maintienne son regard immobile : l'être vivant fixe ce point lumineux avec un œil 21, et la caméra observe l'autre œil 20.

Dans le cadre de ce premier mode de réalisation, à titre d'exemple, on utilise un implant lumineux 1 intracérébral réalisé sous la forme d'une fibre optique implantée dans le cerveau 2, de sorte que l'extrémité de la fibre optique se situe entre les deux substances noires, au niveau du mésencéphale et donc à proximité du chiasma optique. L'implant lumineux 1 a par exemple une puissance crête de 15mW, la lumière étant pulsée avec un rapport cyclique de 8%. Une dilatation de la pupille n'est pas nécessaire. Le noir absolu est fait dans la pièce, et le patient se positionne sur la mentonnière du support.

L'entraxe entre la source de lumière 33 fixe (pour maintenir le regard fixe) et le récepteur 31 (détecteur ou la caméra) est réglé pour correspondre à la distance interoculaire du patient. Dans un exemple particulier, la caméra est une caméra 16 bits, 2750x2200 pixels, binning 2, pixels de 4.54µm, refroidie à -12°C et positionné à environ 5 cm de l'œil. Le temps d'exposition est fixé à 20 secondes. Toutes les sources lumineuses environnantes sont occultées, et l'implant intracérébral est éteint. Comme déjà décrit ci-dessus, le mode de fonctionnement est par exemple le suivant :
- Implant lumineux 1 désactivé, on acquière une première image qui doit être "noire", c'est-à-dire avec uniquement la contribution du bruit interne à la caméra.
- On allume alors l'implant lumineux 1 et on répète l'opération qui met en évidence un spot diffus correspondant aux photons venant de l'implant lumineux 1 intracérébral.

Avantageusement, la source de lumière 33 permettant de fixer le regard émet à une certaine longueur d'onde (vert par exemple, 550nm) tandis que le récepteur 31 (caméra ou détecteur) observant l'autre œil 20 ne sont sensibles qu'à la longueur d'onde d'émission de l'implant lumineux 1 (par exemple au moyen d'un filtre passe haut qui ne laisserait passer que les longueurs d'onde supérieures à 600 nm).

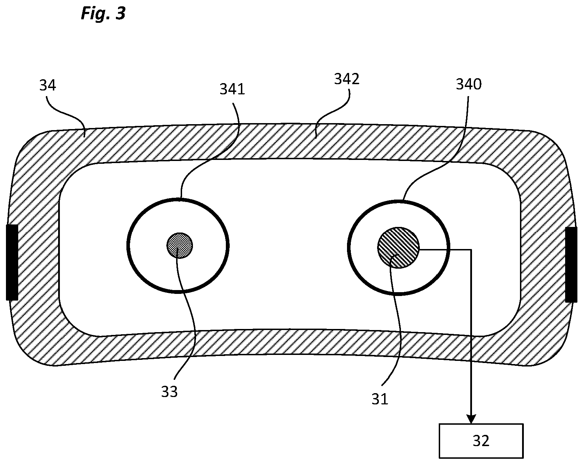

Selon une variante de réalisation représentée sur la figure 3, le support mécanique se présente sous la forme d'un masque 34 à appliquer autour de la tête de l'être vivant.

Le masque 34 porte deux lunettes 340, 341 destinées à être placées respectivement en vis-à-vis des deux yeux 20, 21 de l'être vivant, une lunette portant le récepteur 31 (détecteur ou caméra) et une lunette portant la source de lumière 33 utilisée pour capter le regard de l'être vivant. Le masque 34 comporte des moyens d'occultation 342, utilisés pour créer un canal isolé de toute pollution lumineuse entre le premier œil 20 de l'être vivant et la zone de capture du récepteur 31 et entre la source de lumière 33 et le deuxième œil 21 de l'être vivant.

Cette réalisation peut disposer d'un adaptateur afin de pouvoir intervertir la source de lumière 33 et le récepteur 31, et ainsi pouvoir observer l'œil droit ou l'œil gauche de l'être vivant. Alternativement, on peut avoir sur un même œil, à la fois l'émetteur et le capteur, voire même sur les deux yeux. De plus, l'entraxe entre la source de lumière et le détecteur ponctuel pourrait être réglé afin de pouvoir s'adapter à la distance interoculaire du patient.

Le principe de fonctionnement de ce deuxième mode de réalisation est identique à celui décrit ci-dessus pour le premier mode de réalisation.

Avantageusement, il est possible de réaliser une détection synchrone, afin d'augmenter le rapport signal-bruit : le signal pulsé issu du dispositif de stimulation optique (par exemple détecté électriquement par induction sur le câble d'alimentation du laser, ou optiquement avec un photodétecteur au niveau du crâne, ou numériquement en RF) est alors utilisé comme source externe pour exacerber le signal optique mesuré via l'œil. La source externe peut également venir d'un second détecteur optique posé sur le crâne du patient, au niveau de la source de lumière pulsée, sur le cuir chevelu. Un détecteur mesure le signal diffusé via le cuir chevelu, ce signal jouant le rôle de porteuse pour augmenter le rapport signal à bruit du détecteur observant l'œil, comme pour une détection synchrone.

L'invention présente ainsi de nombreux avantages, parmi lesquels :
- Une facilité de mise en œuvre ;
- Une solution non-invasive ;
- Une solution qui utilise des composant disponibles couramment ;

## Revendications

1. Procédé de détermination de l'état de fonctionnement d'un implant lumineux (1) implanté dans le cerveau (2) d'un être vivant, ledit implant lumineux (1) comportant une source lumineuse (10) chargée d'émettre de la lumière dans le cerveau de l'être vivant, ledit procédé utilisant un dispositif (3) de diagnostic qui comporte un récepteur (31) d'un signal lumineux transmis à travers un premier œil (20) de l'être vivant et des moyens de détermination de l'état de fonctionnement de l'implant lumineux (1) à partir du signal lumineux transmis reçu,
**Caractérisé en ce qu'**il comporte :
- Une étape de positionnement du récepteur (31) du dispositif de diagnostic en vis-à-vis du premier œil (20) de l'être vivant,
- Une étape de mesure d'un signal lumineux reçu par le récepteur (31) lorsque l'implant lumineux (1) est activé,
- Une étape de comparaison entre les données représentatives du signal lumineux reçu par le récepteur (31) et au moins une donnée de référence,
- Une étape de détermination de l'état de fonctionnement de l'implant lumineux (1) entre un état fonctionnel et un état non-fonctionnel en tenant compte du résultat de l'étape de comparaison.

2. Procédé selon la revendication 1, **caractérisé en ce que** ladite donnée de référence est pré-mémorisée ou acquise lors d'une étape de calibration du dispositif (3) de diagnostic.

3. Procédé selon la revendication 2, **caractérisé en ce que** l'étape de calibration consiste en une étape de mesure d'un signal lumineux de référence, réalisée lorsque l'implant lumineux (1) est désactivé.

4. Procédé selon l'une des revendications 1 à 3, **caractérisé en ce que** l'étape de détermination de l'état de fonctionnement de l'implant lumineux (1) consiste à déterminer au moins un écart entre ladite donnée de référence et une donnée desdites données représentatives du signal lumineux reçu et à comparer ledit écart avec une valeur seuil prédéterminée.

5. Procédé selon l'une des revendications 1 à 4, **caractérisé en ce qu'**il comporte une étape de positionnement d'une source de lumière (33) ponctuelle en vis-à-vis d'un deuxième œil (21) de l'être vivant et une étape d'activation de ladite source de lumière (33) ponctuelle.

6. Dispositif (3) de diagnostic utilisé pour mettre en œuvre le procédé tel que défini dans l'une des revendications 1 à 5, **caractérisé en ce qu'**il comporte un support (30, 34) mécanique sur lequel est fixé ledit récepteur (31) et des moyens de détermination (32) de l'état de fonctionnement dudit implant lumineux (1), connectés audit récepteur (31) et configurés pour traiter des données en provenance dudit récepteur.

7. Dispositif selon la revendication 6, **caractérisé en ce que** le récepteur (31) est une caméra.

8. Dispositif selon la revendication 6, **caractérisé en ce que** le récepteur (31) comporte une ou plusieurs photodiodes.

9. Dispositif selon l'une des revendications 6 à 8, **caractérisé en ce qu'**il comporte une source de lumière (33) ponctuelle fixée audit support et destinée à être arrangée en vis-à-vis d'un deuxième œil (21) de l'être vivant, en parallèle du récepteur (31).

10. Dispositif de diagnostic selon la revendication 6, **caractérisé en ce que** le support mécanique comporte un plateau (30) optique muni d'une mentonnière et sur lequel est fixé ledit récepteur (31).

11. Dispositif selon la revendication 6, **caractérisé en ce que** le support mécanique comporte un masque (34) à placer autour de la tête de l'être-vivant.

12. Dispositif selon la revendication 11, **caractérisé en ce que** le masque (34) comporte deux lunettes, une première lunette (340) accueillant ledit récepteur (31) et une deuxième lunette (341) accueillant une source de lumière (33) ponctuelle.

## Patentansprüche

1. Verfahren zur Bestimmung des Betriebszustands eines Lichtimplantats (1), das in das Gehirn (2) eines Lebewesens implantiert ist, wobei das Lichtimplantat (1) eine Lichtquelle (10) umfasst, welche die Aufgabe hat, Licht in das Gehirn des Lebewesens zu emittieren, wobei das Verfahren eine Diagnosevorrichtung (3) einsetzt, die einen Empfänger (31) für ein Lichtsignal umfasst, das durch ein erstes Auge (20) des Lebewesens hindurch übertragen wird, und Mittel zur Bestimmung des Betriebszustands des Lichtimplantats (1) ausgehend von dem empfangenen übertragenen Lichtsignal,
**dadurch gekennzeichnet, dass** es umfasst:
- einen Schritt des Positionierens des Empfängers (31) der Diagnosevorrichtung gegenüber dem ersten Auge (20) des Lebewesens,
- einen Schritt des Messens eines Lichtsignals, das von dem Empfänger (31) empfangen wird, wenn das Lichtimplantat (1) aktiviert ist,
- einen Schritt des Vergleichens zwischen den Daten, die für das von dem Empfänger (31) empfangene Lichtsignal repräsentativ sind, und mindestens einem Referenzdatenelement,
- einen Schritt des Bestimmens des Betriebszustands des Lichtimplantats (1) unter einem betriebsfähigen Zustand und einem nicht betriebsfähigen Zustand unter Berücksichtigung des Ergebnisses des Schritts des Vergleichens.

2. Verfahren nach Anspruch 1, **dadurch gekennzeichnet, dass** das Referenzdatenelement bei einem Schritt des Kalibrierens der Diagnosevorrichtung (3) vorgespeichert oder erfasst wird.

3. Verfahren nach Anspruch 2, **dadurch gekennzeichnet, dass** der Schritt des Kalibrierens in einem Schritt des Messens eines Referenzlichtsignals besteht, der ausgeführt wird, wenn das Lichtimplantat (1) deaktiviert ist.

4. Verfahren nach einem der Ansprüche 1 bis 3, **dadurch gekennzeichnet, dass** der Schritt des Bestimmens des Betriebszustands des Lichtimplantats (1) darin besteht, mindestens eine Abweichung zwischen dem Referenzdatenelement und einem Datenelement der Daten, die für das empfangene Lichtsignal repräsentativ sind, zu bestimmen und die Abweichung mit einem vorbestimmten Schwellenwert zu vergleichen.

5. Verfahren nach einem der Ansprüche 1 bis 4, **dadurch gekennzeichnet, dass** es einen Schritt des Positionierens einer punktförmigen Lichtquelle (33) gegenüber einem zweiten Auge (21) des Lebewesens und einen Schritt des Aktivierens der punktförmigen Lichtquelle (33) umfasst.

6. Diagnosevorrichtung (3), die dazu eingesetzt wird, das in einem der Ansprüche 1 bis 5 definierte Verfahren umzusetzen, **dadurch gekennzeichnet, dass** sie einen mechanischen Halter (30, 34) umfasst, an dem der Empfänger (31) befestigt ist, und Mittel (32) zum Bestimmen des Betriebszustands des Lichtimplantats (1), die mit dem Empfänger (31) verbunden sind und dazu ausgestaltet sind, von dem Empfänger kommende Daten zu verarbeiten.

7. Vorrichtung nach Anspruch 6, **dadurch gekennzeichnet, dass** der Empfänger (31) eine Kamera ist.

8. Vorrichtung nach Anspruch 6, **dadurch gekennzeichnet, dass** der Empfänger (31) eine oder mehrere Fotodioden umfasst.

9. Vorrichtung nach einem der Ansprüche 6 bis 8, **dadurch gekennzeichnet, dass** sie eine punktförmige Lichtquelle (33) umfasst, die an dem Halter befestigt ist und dazu bestimmt ist, gegenüber einem zweiten Auge (21) des Lebewesens, parallel zu dem Empfänger (31), angeordnet zu werden.

10. Diagnosevorrichtung nach Anspruch 6, **dadurch gekennzeichnet, dass** der mechanische Halter eine optische Platte (30) umfasst, die mit einem Kinnriemen versehen ist und an welcher der Empfänger (31) befestigt ist.

11. Vorrichtung nach Anspruch 6, **dadurch gekennzeichnet, dass** der mechanische Halter eine Maske (34) umfasst, die um den Kopf des Lebewesens herum anzulegen ist.

12. Vorrichtung nach Anspruch 11, **dadurch gekennzeichnet, dass** die Maske (34) zwei Lünetten umfasst, eine erste Lünette (340), die den Empfänger (31) aufnimmt, und eine zweite Lünette (341), die eine punktförmige Lichtquelle (33) aufnimmt.

## Claims

1. Method for determining the operating state of a light-emitting implant (1) implanted in the brain (2) of a living being, said light-emitting implant (1) comprising a light source (10) responsible for emitting light into the brain of the living being, said method using a diagnosing device (3) that comprises a receiver (31) of a light signal transmitted through a first eye (20) of the living being and means for determining the operating state of the light-emitting implant (1) based on the received transmitted light signal,
**characterized in that** it comprises:
- a step of positioning the receiver (31) of the diagnosing device facing the first eye (20) of the living being,
- a step of measuring a light signal received by the receiver (31) when the light-emitting implant (1) is activated,
- a step of comparing data representative of the light signal received by the receiver (31) and at least one reference datum,
- a step of determining the operating state of the light-emitting implant (1) between a functional state and a non-functional state given the result of the comparing step.

2. Method according to Claim 1, **characterized in that** said reference datum is stored beforehand or acquired in a step of calibrating the diagnosing device (3).

3. Method according to Claim 2, **characterized in that** the calibrating step consists in a step of measuring a reference light signal, carried out when the light-emitting implant (1) is deactivated.

4. Method according to one of Claims 1 to 3, **characterized in that** the step of determining the operating state of the light-emitting implant (1) consists in determining at least one difference between said reference datum and a datum of said data representative of the received light signal and in comparing said difference with a predetermined threshold value.

5. Method according to one of Claims 1 to 4, **characterized in that** it comprises a step of positioning a point light source (33) facing a second eye (21) of the living being and a step of activating said point light source (33).

6. Diagnosing device (3) used to implement the method such as defined in one of Claims 1 to 5, **characterized in that** it comprises a mechanical mount (30, 34) to which said receiver (31) is fastened and means (32) for determining the operating state of said light-emitting implant (1), said means being connected to said receiver (31) and configured to process data delivered by said receiver.

7. Device according to Claim 6, **characterized in that** the receiver (31) is a camera.

8. Device according to Claim 6, **characterized in that** the receiver (31) comprises one or more photodiodes.

9. Device according to one of Claims 6 to 8, **characterized in that** it comprises a point light source (33) fastened to said mount and intended to be arranged facing a second eye (21) of the living being, in parallel with the receiver (31).

10. Diagnosing device according to Claim 6, **characterized in that** the mechanical mount comprises an optical platform (30) provided with a chin rest, and to which said receiver (31) is fastened.

11. Device according to Claim 6, **characterized in that** the mechanical mount comprises a headset (34) to be placed around the head of the living being.

12. Device according to Claim 11, **characterized in that** the headset (34) comprises two eyeglasses, a first eyeglass (340) accommodating said receiver (31) and a second eyeglass (341) accommodating a point light source (33).
